(19) Europäisches Patentamt
European Patent Office
Office européen des brevets

(11) Publication number: 0 217 316 B1

# (12) EUROPEAN PATENT SPECIFICATION

(45) Date of publication of patent specification: 17.05.89

(51) Int. Cl.[4]: C 07 C 25/22, C 07 C 17/00

(21) Application number: 86113297.5

(22) Date of filing: 26.09.86

## (54) Process for producing brominated acenaphthylene and its condensates.

(30) Priority: 27.09.85 JP 212296/85

(43) Date of publication of application:
08.04.87 Bulletin 87/15

(45) Publication of the grant of the patent:
17.05.89 Bulletin 89/20

(84) Designated Contracting States:
BE DE FR GB IT

(56) References cited:
EP-A-0 128 465
DE-A-3 335 400

PATENT ABSTRACTS OF JAPAN, unexamined applications, Field C, vol. 9, no. 237, September 24, 1985, THE PATENT OFFICE JAPANESE GOVERNMENT, p. 71 C 305

(73) Proprietor: TOSOH CORPORATION
4560, Oaza-Tonda Shinnanyo-shi
Yamaguchi 746 (JP)

(72) Inventor: Kubo, Masashige
6772, Oaza Tokuyama
Tokuyama-shi Yamaguchi-ken (JP)
Inventor: Kawabata, Koji
2515-23, Oaza Fukugawa
Shinnanyo-shi Yamaguchi-ken (JP)
Inventor: Tsutsumi, Yukihiro
1723-12, Oaza Shimokami
Tokuyama-shi Yamaguchi-ken (JP)

(74) Representative: Vossius & Partner
Siebertstrasse 4 P.O. Box 86 07 67
D-8000 München 86 (DE)

EP 0 217 316 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a process for separating and recovering brominated acenaphthylene and its condensates in a powdery form from their solution obtained in the course of production.

Brominated acenaphthylene and its condensates (designated hereinafter as Con-BACN) are compounds having excellent uncombustive and radiation-resistive properties and, when they are admixed to various kinds of resins, they can donate to the resins the uncombustive and radiation-resistive properties. Due to the double bonds in their molecules, they can be grafted to a resin by inciting them to generate free radicals. As condensates they have a good immiscibility with resins and therefore they are capable of maintaining stable their uncombustive and radiation-resistive properties for a long period of time (seen US—A—4,373,046). As a result thereof, Con-BACN can be used in atomic reactors, breeder reactors and ionization radiation generators as insulating materials for coating cables and various resin compositions which require radiation resistance as well as uncombustive property. The Con-BACN of this invention contains at least one bromine atom on the aromatic ring which is produced by condensation as a result of formal Friedel-Crafts' reaction of brominated acenaphthene to form polymers of a degree of condensation of 2 or more followed by dehydrobromination, including the brominated acenaphthylene produced by the dehydrobromination of brominated acenaphthene without being condensed.

The Con-BACN of this invention is expressed by a general formula (I);

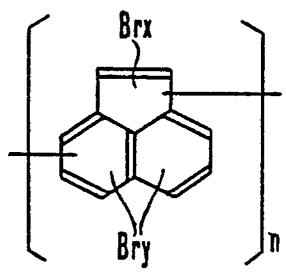

(I)

wherein x is an integer of 0—1, y is an integer of 1—6, and n is an integer of 1 or more. The bond is formed between carbon atoms at the benzyl and the aryl positions of acenaphthylene, for example:

1(or 2), 5' -

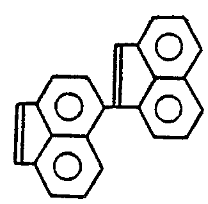

1(or 2), 6' -

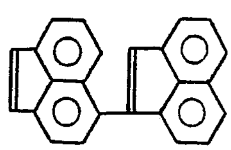

Otherwise, there may be bonds such as, for example, 1(or 2), 3' —, 1(or 2), 4' —, 1(or 2), 7' —, and 1(or 2), 8' —. Condensates having a degree of condensation of 3 or more contain any of the bonds mentioned above to increase the number of constituents. The condensates referred to in the present invention are those having a degree of condensation of 10 or less and being excellent in the immiscibility with resins.

Con-BACN is usually produced in a powdery form, but when recovered by the separation method, resinous conglomerates are obtained. Since the compound is intended to be used in a composition with a resin or rubber, the finely divided powdery form is favored for convenience of handling and easy dispersion when admixed to a resin composition.

Con-BACN is generally produced by the bromination and condensation of acenaphthene followed by dehydrobromination, i.e. bromine is added to acenaphthene in a halogenated hydrocarbon solvent in the presence of a Lewis catalyst for bromination and condensation, and the halogenated acenaphthene condensate obtained is treated with a base such as potasium hydroxide in a methanolic solution for dehydrobromination. Dehydrobromination is carried out in a solvent which is inactive to a base, such as

potassium hydroxide in methanol. Thus, the solvents include halogenated and aromatic hydrocarbon solvents. Therefore, the produced Con-BACN is obtained in the form of a solution in a good solvent, namely halogenated or aromatic hydrocarbon. For the purpose of recovering Con-BACN in a powdery form by separation from a solution of Con-BACN, methods of reprecipitation are commonly practiced, where the solution of Con-BACN is added to a poor solvent in which Con-BACN has a slight solubility. They include, for example, a method of reprecipitation in acetone (Y. Morita and M. Hagiwara, J. Appl. Polym. Sci., *27*, 3329 (1982)), a method of reprecipitation which some of the present inventors previously disclosed in EP—A—128 465 using monohydric aliphatic alcohols containing 3 to 5 carbon atoms for the poor solvent and a method of reprecipitation disclosed in EP—A—128 465 which uses, as poor solvent, saturated aliphatic hydrocarbons containing 5 to 9 carbon atoms.

In these methods Con-BACN can be obtained in the form of a powder, but the methods are not free from drawbacks. Particularly, after the reprecipitation procedure, Con-BACN of low degrees of condensation remains dissolved in the filtrate, hence the recovery rate of Con-BACN is lowered. Furthermore, the condensation composition of Con-BACN is somewhat different before and after reprecipitation and, at the same time, the physical properties of the powder are accordingly varied, resulting in problems in the quality control. An additional problem is the separation and recovery of the good and poor solvents remaining dissolved in the filtrate after reprecipitation and treatment of the Con-BACN.

For the purpose of solving these problems, some of the present inventors attempted to develop a process in which saturated aliphatic hydrocarbons were used as poor solvent to reprecipitate Con-BACN, the slurry obtained was distilled to recover the good solvent, powders of Con-BACN were separated and the filtrate was circulatingly used in the subsequent reprecipitation. This was applied for patent EP—A—128 465.

A good result was obtained by the separation and recovery method of the above-mentioned application, but the method was not free from troubles in operation.

In addition, there also existed troubles regarding working environment and safe handling, because inflammable organic solvents were involved in mixing, stirring, filtering and drying. Thus, this method was not satisfactorily employed as an industrial method for separation and recovery.

While the previous processes of separating and recovering powdery Con-BACN were troublesome in operation and involved economical problems as mentioned above, the object of the present invention is to provide an economically profitable industrial process by simplifying existing processes.

A further object is to develop an industrial process in which the safety in operation is enhanced by keeping workers away from exposure to organic solvents and avoiding inflammation and explosion of inflammable organic solvents.

With the problems of previous processes in mind, the present inventors were concerned with the process in which a solution of Con-BACN is added dropwise to heated water to disperse Con-BACN in the water in the form of a powder for separation and recovery while the solvent is being removed. In intensive investigations it was found that the strong hydrophobic property of Con-BACN prevents the Con-BACN to be obtained in the form of finely divided powder, because a viscous condensate coagulates while the solvent is being removed, and adheres to the inside wall of a tank and to the blades of a stirrer, to form conglomerates without forming powders of Con-BACN. This naturally brings about difficulties of operation.

For solving this problem, the present inventors searched for a dispersing agent of Con-BACN in water. It was found that a variety of cationic, anionic and nonionic surfactants do not exhibit a dispersing effect when they are added in a small amount, but, if applied in a larger amount to improve the dispersion, they bubble vigorously when the solvent is stripped to such a magnitude as to prohibit satisfactory continuation of the operation.

When the amount of the dispersing agent is increased, different kinds of problems arise; disturbance of the dispersing agent remaining in Con-BACN, pollution of the factory exhausts, and the cost of product increase due to the necessary cost of dispersing agents. Therefore, a dispersing agent is desired which in a slight amount is capable of producing fine powders of Con-BACN.

Further search for dispersing agents revealed that among the nonionic surfactants polyoxyalkylene sorbitan carboxylic acid esters were effective in a very small amount to disperse Con-BACN in water and to form finely divided particles. In addition, less bubbles were formed when the solvent was removed. Accordingly good operatability of the process accelerated the completion of the present invention. In short, the gist of this invention consists in the manufacturing process of Con-BACN comprising dropwise adding a solution of the Con-BACN obtained in the course of production to hot water containing a polyoxyalkylene sorbitan carboxylic acid ester, while the solvent is distilled off, to recover Con-BACN in the form of a powder dispersed in water.

Details of the invention will be explained below.

The separation and recovery of Con-BACN of this invention can be attained by dropwise adding a solution of the Con-BACN obtained in the course of production to hot water containing polyoxyalkylene sorbitan carboxylic acid ester to obtain the Con-BACN in the form of powders dispersed in water while distilling off the solvent.

The organic solvents referred to in this invention which are used for the Con-BACN obtained in the

course of production are the good solvents to dissolve Con-BACN including those halogenated hydrocarbons and aromatic hydrocarbons which are inactive in dehydrobromination and either boil alone or azeotropically boil with water at a temperature below 100°C. They are for example carbon tetrachloride, chloroform, methylene chloride, ethylenedichloride, ethylenedibromide, chlorobenzene, benzene, toluene, xylene and ethylbenzene.

There is no substantial restriction on the concentration of Con-BACN solution, but usually a concentration of 5—70% by weight is preferred.

The polyoxyalkylene sorbitan carboxylic acid esters to be used in the present invention are obtained by converting a mixture of 1,5-sorbitan, 1,4-sorbitan, 3,6-sorbitan, 1,4,3,6-sorbide obtained by intramolecular dehydration of sorbitol into partially esterified derivatives of carboxylic acid esters, and by combining nonreacting hydroxyl groups with polyoxyalkylene chains. They are expressed by general formulae [II], [III] and [IV] or a mixture thereof.

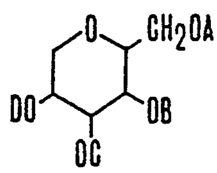
(II)

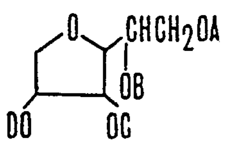
(III)

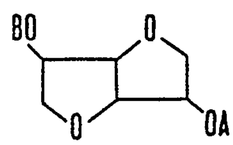
(IV)

wherein A is an acyl group and B, C and D are acyl groups or polyoxyalkylene groups expressed by the general formula $(C_lH_{2l}O)_mH$ (l is an integer of 2—4 and m is an integer of 1—50), and at least one of the substituting groups for each compound is a polyoxyalkylene group. For the carboxylic acid part of these compounds are selected those straight chain carboxylic acids containing 10—18 carbon atoms, for example, lauric, palmitic, stearic and oleic acids.

Further, the HLB (hydrophilic and lipophilic balance) value of these compounds is in the range from 8 to 18.

The polyoxyalkylene sorbitan carboxylic acid esters of this invention include, for example, polyoxyethylene sorbitan monolaurate, polyoxyethylene sorbitan monopalmitate, polyoxyethylene sorbitan monostearate, polyoxyethylene sorbitan tristearate, polyoxyethylene sorbitan monooleate, polyoxyethylene sorbitan trioleate and polyoxypropylene sorbitan monolaurate. The amount of these surfactants to be applied differs somewhat depending on the working conditions. However, in most cases, it is usually 0.01 to 5 parts, preferably 0.05 to 1 part by weight per 100 parts by weight of Con-BACN powder.

When the amount of the surfactants is less than 0.01 part by weight, the Con-BACN deposited by removal of the solvent does not disperse in good condition in the hot water, but partly coagulates, thus not forming a uniformly dispersed powdery matter.

When the amount of the surfactants exceed 5 parts by weight, bubbles evolving at the solvent removal become somewhat vigorous, thus creating an inferior condition of operation and the defect mentioned above.

The surfactants are usually mixed beforehand in the hot water, but it may partially be mixed into drops of the Con-BACN solution for easy operation.

The amount of hot water in the tank where the removal of the solvent takes place is determined according to the concentration of slurry of Con-BACN obtained after the solvent has been removed. The amount should be selected from the standpoint of easy handling and cost. Usually 0.2 to 10 liter of hot water per 100 g of Con-BACN are employed.

The temperature of the hot water in the solvent removal tank should be higher than the boiling point of solvent of the Con-BACN solution or the temperature of the azeotropic mixture with water and it usually is approximately 40—100°C. While the solvent is being removed, the liquid in the solvent removal tank should be stirred thoroughly so as to obtain a uniform dispersion of Con-BACN in the water. For this purpose the solution of Con-BACN should be added to the hot water either by dropping it onto the surface of the water or by injecting it through a nozzle of a small diameter.

The speed of dropping the Con-BACN solution into the hot water should preferably be less than the distilling speed of the organic solvent in order to prevent solidification of the Con-BACN. Preferably, the speed is 0.01—0.3 l/hr per liter of hot water.

The process of the present invention is usually carried out under normal pressure, but it can also be conducted under reduced pressure.

When the Con-BACN solution is added in drops to hot water which contains the above-mentioned surfactants, and the solvent is removed at the same time by the mentioned procedure, Con-BACN is separated in a short period, forming finely divided power uniformly dispersed in the water.

The powders of Con-BACN thus deposited can easily be separated from the slurry by a conventional method, for example by centrifugation, filtration under suction and drying by spraying.

By using these methods, Con-BACN can be separated and recovered quantitatively in the form of a powder from the reaction liquid obtained in the course of production.

As has been clearly explained above, the process of the present invention permits the Con-BACN powder to be separated and recovered from water.

By the process of this invention, therefore, the Con-BACN powder could be quantitatively separated and recovered with more ease than in the previous reprecipitation method employing organic solvents, hence with more ease in the production control.

Those previous problems which are concerned with the recovery of the Con-BACN remaining dissolved in the filtrate and with the separation for recovering the solvent have been rendered unnecessary. This permits the process to be simplified to a large extent. The working environment has considerably been improved to better hygienic conditions for workers by reducing the time of exposure to organic solvents. Higher safety in operation can be obtained since e.g. the stirring, mixing, filtering and drying of inflammable organic solvents is no longer required.

The powders of Con-BACN obtained by the process of this invention have the same order of particle size as those produced by the reprecipitation method using an organic solvent. Therefore they can easily be dispersed when admixed to compositions of resins or rubber.

A Con-BACN powder prepared by the reprecipitation method with an organic solvent as precipitant often contains the organic solvent by inclusion in the particles. The solvent can hardly be removed by an ordinary drying procedure. However, with the Con-BACN powder prepared by the process of this invention an organic solvent can easily be removed in an ordinary drying procedure without any additional purification process. High thermal stability is another feature of the Con-BACN powders of this invention.

In the process of the present invention, Con-BACN powder of high quality can be separated and recovered with industrial profit in a more simplified process than previous ones.

Detailed Description of the Preferred Embodiments:

The process of the present invention will be described in detail with the following embodiments, but the invention is not limited to them.

Example 1

A mixture of 463 g of acenaphthene and 4.92 g of 2,2'-azo-bis(isobutylonitrile) was added to 1400 ml of carbon tetrachloride and the resulting solution was refluxed at 77°C. To this solution was added 312 g of bromine in solution in 460 ml of carbon tetrachloride under stirring in 0.9 hr and the reaction was continued for 0.5 hr. Then the reaction solution was cooled, 950 ml of carbon tetrachloride was added, followed by adding 28.5 g of titanium tetrachloride at 25°C, and the reaction was continued for one more hour. Subsequently, 8.4 g of iron powder was added to the reacting solution and 2300 g of bromine was dropwise added in 3.5 hr. The temperature was gradually elevated and the mixture was refluxed under heating for 2 hr. At the completion of the reaction, an aqueous solution of sodium hydrogen sulfite was added to remove excessive bromine and, after insoluble matter was removed by filtration, the reaction solution was thoroughly washed with water.

A solution of 336 g of potassium hydroxide in 1300 ml of methanol was added to the reaction solution, and the mixture was refluxed to react under heating for 2 hr.

The potassium bromide deposited was separated by filtration and washed with water to remove methanol, to obtain 3 l of a solution which contained 960 g of Con-BACN and 2.6 l of carbon tetrachloride.

Analysis showed that the Con-BACN contained 63.3% of bromine, and the condensation composition as found by high performance liquid chromatography was 18.0% of monomer, 37.6% of dimer, 27.0% of trimer and 17.4% of tetra — to octomer. Hereinafter the carbon tetrachloride solution containing Con-BACN is called 'treatment solution'. A 0.5 l aliquot of the 'treatment solution' containing 160 g of Con-BACN was employed for the following separation and recovery.

Polyoxyethylene sorbitan monopalmitate (trade name: Leodol TW—D120, supplied from Kao Soap Co. Ltd.) in an amount of 0.48 g, having an HLB value of 15.6, was dissolved in 1.5 l water and heated at 95°C. To this heated water, the above 'treatment solution' was added in drops and under stirring at a speed of 0.2 l/hr. The carbon tetrachloride was continuously distilled off. During distillation hardly any bubbling took place and Con-BACN was immediately deposited to form fine powders which dispersed uniformly in the water. When the dropwise addition of the 'treatment solution' was finished, an aqueous slurry of Con-BACN obtained was filtered, the powder was washed with 3 l of water and dried for 8 hr at 120°C to obtain 159.7 g of reddish orange powder of Con-BACN.

Recovery of Con-BACN from the 'treatment solution' took place quantitatively. The results obtained and the analysis of the Con-BACN powder are shown in Table 1.

Example 2

In 2.5 l water, 0.4 g of polyoxyethylene sorbitan monolaurate (trade name: Tween 20 supplied from Wako Pure Chemicals Co. Ltd.) having an HLB value of 16.7 was dissolved and heated to 90°C. In a 0.5 l aliquot from the 'treatment solution' prepared in Example 1 which contained 160 g of Con-BACN, 0.4 g of above-mentioned polyoxyethylene sorbitan monolaurate was dissolved and the solution obtained was

dropwise added to the above heated water at a speed of 0.1 l/hr, while carbon tetrachloride was continuously being distilled. Hardly any bubbling was observed during the distillation and the Con-BACN deposited immediately formed a fine powder and dispersed uniformly in the water.

At the completion of dropwise addition of the 'treatment solution', an aqueous slurry containing the Con-BACN obtained was treated in the same manner as in Example 1, to obtain a fine powder of Con-BACN in reddish orange color. The results obtained and the analysis of the Con-BACN powder are shown in Table 1.

### Example 3

In 2 l water, 0.36 g of polyoxyethylene sorbitan monostearate (trade name: Tween 60 supplied from Wako Pure Chemicals Co. Ltd.) having an HLB value of 14.9 was dissolved and heated to 95°C. In a 0.5 l aliquot from the 'treatment solution' prepared in Example 1 which contained 160 g of Con-BACN was dropwise added to the above heated stirred water at a speed of 0.2 l/hr, while carbon tetrachloride was continuously being distilled. Hardly any bubbling was observed during the distillation and the Con-BACN deposited immediately formed a fine powder and dispersed uniformly in the water. The sequence of the procedure took place very smoothly.

At the completion of dropwise addition of the 'treatment solution', an aqueous slurry containing the Con-BACN obtained was treated in the same manner as in Example 1, to obtain a fine powder of Con-BACN in reddish orange color. The results obtained and the analysis of the Con-BACN powder are shown in Table 1.

### Example 4

In 1.5 l water, 0.48 g of polyoxyethylene sorbitan trioleate (trade name: Leodol TW—O—320 supplied from Kao Soap Co. Ltd.) having an HLB value of 11.0 was dissolved and heated to 95°C. A 0.5 l aliquot from the 'treatment solution' prepared in Example 1 containing 160 g of Con-BACN, of which the solvent carbon tetrachloride was replaced by benzene so as to maintain the same solution volume, was dropwise added to the above heated stirred water at a speed of 0.2 l/hr, while the solvent benzene was continuously being distilled. Hardly any bubbling was observed during the distillation and the Con-BACN deposited immediately formed a fine powder and dispersed uniformly in the water. The sequence of the procedure took place very smoothly.

At the completion of dropwise addition of the 'treatment solution', an aqueous slurry containing the Con-BACN obtained was treated in the same manner as in Example 1, to obtain a fine powder of Con-BACN in reddish orange color. The results obtained and the analysis of the Con-BACN powder are shown in Table 1.

### Comparison Example 1

A 0.5 l aliquot of the 'treatment solution' in carbon tetrachloride prepared in Example 1 containing 160 g of Con-BACN was added in drops to 2 l of cold (0 — −10°C) acetone at a speed of 0.2 l/hr under stirring. At the completion of the dropwise addition, the powder deposited was separated by filtration and dried at 120°C for 8 hr, to obtain a fine powder of Con-BACN in reddish orange color. The results obtained and the analysis of Con-BACN powder are shown in Table 1.

### Comparison Example 2

A 0.5 l aliquot of the 'treatment solution' in carbon tetrachloride prepared in Example 1 containing 160 g of Con-BACN was added in drops to 2 l of i-Octane at room temperature at a speed of 0.2 l/hr under stirring. At the completion of the dropwise addition, the powder deposited was separated by filtration and dried at 120°C for 8 hr, to obtain a fine powder of Con-BACN in reddish orange color. The results obtained and the analysis of Con-BACN powder are shown in Table 1.

### Comparison Example 3

A 1.5 l water, 0.5 g of sodium stearate was dissolved and the solution was heated to 95°C. Into a 0.5 l aliquot from the 'treatment solution' in carbon tetrachloride prepared in Example 1 containing 160 g of Con-BACN was dropwise added to the above-mentioned heated water at a speed of 0.2 l/hr under stirring, while carbon tetrachloride was continuously being distilled. Vigorous bubbling occurred during the distillation which made it difficult to continue the process and the Con-BACN deposited harldy dispersed in water, but mostly adhered to the stirrer blades and the inner wall of vessel in a resinous form, so that the Con-BACN could not be obtained in the form of powder.

### Comparison Example 4

The same charging of materials and procedures as in Comparison Example 3 were employed except that the surfactant was changed to stearyltrimethylammonium chloride (trade name: Cortamine 86P conc, supplied from Kao Soap Co. Ltd.). The separation and recovery of Con-BACN was not successful because of vigorous bubbling. The Con-BACN deposited adhered as a resinous matter to the stirrer blades and to the inner wall of the vessel. Con-BACN powder could not be obtained.

Comparison Example 5

In 1.5 l water, 0.8 g of polyoxyethylene nonylphenyl ether (trade name: Nonypol 160 supplied from Sanyo Chemical Industry Ltd.) having an HLB value of 15.2 was dissolved and heated to 95°C. A 0.5 l aliquot from the 'treatment solution' in carbon tetrachloride prepared in Example 1 containing 160 g of Con-BACN was dropwise added to the above-mentioned heated water at a speed of 0.1 l/hr under stirring, while the solvent carbon tetrachloride was continuously being distilled. Vigorous bubbling took place during the distillation, and the process became difficult to continue. The Con-BACN deposited formed a resinous matter floating on the surface of the water and therefore Con-BACN could not be obtained in the form of a powder.

Comparison Example 6

Separation and recovery of Con-BACN were attempted with the same charging and procedures as in Comparison Example 5, except that the surfactant was changed to sorbitan monopalmitate (trade name: Span 40 supplied for Wako Pure Chemicals Co. Ltd.) having an HLB value of 6.7. The Con-BACN deposited coagulated into a resinous matter without being dispersed in hot water at all. The stirrer could not be operated and the process could not possibly be proceeded any further.

Table 1

| Result and analysis of powder \ Example | Example 1 | Example 2 | Example 3 | Example 4 | Comparison Example 1 | Comparison Example 2 |
|---|---|---|---|---|---|---|
| Result | | | | | | |
| Yield of Con-BACN (g) | 159.7 | 159.5 | 159.7 | 159.8 | 112.0 | 131.2 |
| Recovery of Con-BACN (%) | 99.8 | 99.7 | 99.8 | 99.9 | 70.0 | 82.0 |
| Mean particle diameter (μm) (1) | 8.9 | 7.6 | 8.3 | 8.7 | 9.6 | 11.3 |
| Melting point (°C) | 144-153 | 143-153 | 144-153 | 144-153 | 152-163 | 148-160 |
| Condensation composition (HLC Ar %) (2) | | | | | | |
| Monomer | 17.9 | 18.0 | 18.0 | 17.9 | 12.7 | 14.5 |
| Dimer | 37.7 | 37.5 | 37.7 | 37.6 | 34.5 | 36.2 |
| Trimer | 27.0 | 27.1 | 27.0 | 27.1 | 29.5 | 28.3 |
| Tetramer - Octomer | 17.4 | 17.4 | 17.3 | 17.4 | 23.3 | 21.0 |
| Solvent content (% wt) (3) | | | | | | |
| Carbon tetrachloride | < 0.01 | < 0.01 | < 0.01 | - | 1.21 | 0.77 |
| Benzene | - | - | - | < 0.01 | - | - |
| Others | - | - | - | - | Acetone < 0.01 | i-Octane 1.36 |
| Thermal decomposition (% wt) (4) | 0.02 | 0.03 | 0.03 | 0.02 | 0.09 | 0.10 |

Remarks

(1) The value at 50% of the particle size distribution estimated with the Coulter Counter Model TAII (manufactured by Coulter Electronics Corp.) and aperture tubes of 140 μm.

(2) Analysis made by high performance liquid chromatography,

Apparatus: High Performance Liquid Chromatograph, Model TSK HLC 802 from Toyo Soda Manufacturing Co., Ltd.

Column: 7.5 mm diameter and 600 mm length.

Liquid: TSK GEL G1000H8 from Toyo Soda Manufacturing Co., Ltd.

(3) Analysis made by gas chromatography.

(4) Estimated from the amount of hydrogen bromide gas evolved by heating Con-BACN powders for 3 hr at 160°C in a nitrogen atmosphere.

**Claims**

1. A process for recovering brominated acenaphthylene and its condensates in the form of a powder, comprising dropping a solution of brominated acenaphthylene and its condensates produced from acenaphthene

by bromination, condensation and dehydrobromination into hot water containing a carboxylic acid ester of a polyoxyalkylene sorbitan, while the solvent is being distilled off, to recover the brominated acenaphthylene and its condensates in the form a powder dispersed in water.

2. A process according to claim 1, wherein the solvent for the brominated acenaphthylene and its condensates is a halogenated or aromatic hydrocarbon which can be distilled or azeotropically distilled with water at a temperature below 100°C.

3. A process according to claim 1, wherein the carboxylic acid ester of polyoxyalkylene sorbitan is used in an amount ranging from 0.01 to 5% by weight relative to the amount of brominated acenaphthylene and its condensates.

4. A process according to claim 1, wherein the carboxylic acid ester of polyoxyalkylene sorbitan is used in an amount ranging from 0.05 to 5% by weight relative to the amount of brominated acenaphthylene and its condensates.

5. A process according to claim 1, wherein the carboxylic acid ester of polyoxyalkylene sorbitan has an HLB value ranging from 8 to 18.

6. A process according to claim 1, wherein the carboxylic acid ester of polyoxyalkylene sorbitan is polyoxyethylene sorbitan monolaurate, polyoxyethylene sorbitan monopalmitate, polyoxyethylene sorbitan monostearate, polyoxyethylene sorbitan tristearate, polyoxyethylene sorbitan monooleate, polyoxyethylene sorbitan trioleate or polyoxypropylene sorbitan monolaurate.

7. A process according to claim 1, wherein the hot water has a temperature from about 40 to about 100°C.

**Patentansprüche**

1. Verfahren zur Gewinnung von bromierten Acenaphthylen und seinen Kondensation in Form eines Pulvers durch Eintropfen einer Lösung von bromierten Acenaphthylen oder seinen Kondensation, hergestellt aus Acenaphthen durch Bromierung, Kondensation und Dehydrobromierung, in einen Carbonsäureester eines Polyoxyalkylensorbitan enthaltendes heißes Wasser, wobei das Lösungsmittel abdestilliert wird, und Gewinnung des bromierten Acenaphthylens und seiner Kondensate in Form eines in Wasser dispergierten Pulvers.

2. Verfahren nach Anspruch 1, in dem das Lösungsmittel für das bromierte Acenaphthylen und seine Kondensate ein halogenierter oder aromatischer Kohlenwasserstoff ist, der bei einer Temperatur unter 100°C mit Wasser destilliert oder azeotrop destilliert werden kann.

3. Verfahren nach Anspruch 1, in dem der Carbonsäureester des Polyoxyalkylensorbitans in einer Menge von 0,01 bis 5 Gew.-%, bezogen auf die Menge des bromierten Acenaphthylens oder seiner Kondensate verwendet wird.

4. Verfahren nach Anspruch 1, in dem der Carbonsäureester des Polyoxyalkylensorbitans in einer Menge von 0,05 bis 5 Gew.-%, bezogen auf die Menge des bromierten Acenaphthylens oder seiner Kondensate verwendet wird.

5. Verfahren nach Anspruch 1, in dem der Carbonsäureester des Polyoxyalkylensorbitans einen HLB-Wert von 8 bis 18 aufweist.

6. Verfahren nach Anspruch 1, in dem der Carbonsäureester des Polyoxyalkylensorbitans Polyoxyäthylensorbitanmonolaurat, Polyoxyäthylensorbitanmonopalmitat, Polyoxyäthylensorbitanmonostearat, Polyoxyäthylensorbitantristearat, Polyoxyäthylensorbitanmonooleat, Polyoxyäthylensorbitantrioleat oder Polyoxypropylensorbitanmonolaurat ist.

7. Verfahren nach Anspruch 1, in dem das heiße Wasser eine Temperatur von etwa 40 bis etwa 100°C aufweist.

**Revendications**

1. Procédé de récupération d'acénaphtylène bromé et de ses produits de condensation sous la forme d'une poudre, comprenant l'addition goutte à goutte d'une solution d'acénaphtylène bromé et de ses produits de condensation obtenus à partir d'acénaphtène par bromation, condensation et déshydrobromation dans de l'eau chaude contenant un ester d'acide carboxylique d'un polyoxcyalkylène sorbitane, en éliminant le solvant par distillation, pour récupérer l'acénaphtylène bromé et ses produits de condensation sous la forme d'une poudre dispersée dans l'eau.

2. Procédé selon la revendication 1, dans lequel le solvant pour l'acénaphtylène bromé et ses produits de condensation est un hydrocarbure halogéné ou aromatique que l'on peut distiller ou distiller par distillation azéotropique avec l'eau à une température inférieure à 100°C.

3. Procédé selon la revendication 1, dans. lequel on utilise l'ester d'acide carboxylique de polyoxyalkylènesorbitane en une quantité comprise entre 0,01 et 5% en poids par rapport à la quantité d'acénaphtylène bromé et de ses produits de condensation.

4. Procédé selon la revendication 1, dans lequel on utilise l'ester d'acide carboxylique de polyoxyalkylène sorbitane en une quantité comprise entre 0,05 et 5% en poids par rapport à la quantité d'acénaphtylène bromé et de ses produits de condensation.

5. Procédé selon la revendication 1, dans lequel l'ester d'acide carboxylique de polyoxyalkylène sorbitane présente un rapport hydrophile/lipophile compris entre 8 et 18.

6. Procédé selon la revendication 1, dans lequel l'ester d'acide carboxylique de polyoxyalkylène sorbitane est le monolaurate de polyoxyéthylène sorbitane, le monopalmitate de polyoxyéthylène sorbitane, le monostéarate de polyoxyéthylène sorbitane, le tristéarate de polyoxyéthylène sorbitane, le monooléate de polyoxyéthylène sorbitane, le trioléate de polyoxyéthylène sorbitane ou le monolaurate de polyoxypropylène sorbitane.

7. Procédé selon la revendication 1, dans lequel l'eau chaude a une température comprise entre environ 40 et environ 100°C.